(19) Europäisches Patentamt
European Patent Office
Office européen des brevets

(11) Publication number: **0 393 530 B1**

(12) # EUROPEAN PATENT SPECIFICATION

(45) Date of publication of patent specification: **28.12.94**

(51) Int. Cl.5: **C07D 471/04**, A61K 31/55, //(C07D471/04,243:00,221:00)

(21) Application number: **90107099.5**

(22) Date of filing: **12.04.90**

(54) **5,11-dihydro-6H-pyrido[2,3,-b][1,4]benzodiazepin-6-ones and -thiones and their use in the prevention or treatment of AIDS.**

(30) Priority: **20.04.89 US 340937**
**28.06.89 US 372728**
**17.11.89 US 438922**

(43) Date of publication of application:
**24.10.90 Bulletin 90/43**

(45) Publication of the grant of the patent:
**28.12.94 Bulletin 94/52**

(84) Designated Contracting States:
**AT BE CH DE DK ES FR GB GR IT LI LU NL SE**

(56) References cited:
**DE-A- 1 966 815**
**FR-A- 1 542 160**

**Chemical Abstracts, vol. 90, no.17, April 23, 1979 Columbus Ohio, USA THOMAE, DR. KARL, G.M.B.H." 11-Substituted 5,11-dihydro-6H-pyrido-(2,3-b)(1,4) benzodiazepin-6-ones." & Belg. 867,639**

(73) Proprietor: **BOEHRINGER INGELHEIM PHARMACEUTICALS INC.**
**900 Ridgebury Road,**
**P.O. Box 368**
**Ridgefield,**
**Connecticut 06877-0368 (US)**

Proprietor: **Dr. Karl Thomae GmbH**

**D-88397 Biberach (DE)**

(72) Inventor: **Hargrave, Karl D., Dr.**
**4 Edna Drive**
**Brookfield, Connecticut 06805 (US)**
Inventor: **Schmidt, Günther, Dr.**
**deceased on**
**August 08, 1986 (DE)**
Inventor: **Engel, Wolfhard, Dr.**
**Mozartstrasse 13**
**W-7950 Biberach 1 (DE)**
Inventor: **Austel, Volkhard, Dr.**
**Kapellenweg 7**
**W-7950 Biberach 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 393 530 B1

CHEMICAL ABSTRACTS, vol. 82, no.25, june 23, 1975 Columbus, Ohio, USA VON BEBENBURG, WALTER " Aza-10, 11-dihydro-5H-dibenzo(b,e)-(1,4)diazepines."&S. African 73 07,643

CHEMICAL ABSTRACTS, vol. 73, no. 15, OCTOBER 12, 1970 Columbus, OHIO, USA SCHIMIDT, GUENTHER et al. "Physiologically active 5,11-dihydro-6H-Pyrido (2,3-b)-(1,4)benzodiazepin-6-ones." & S. African 69 05,933

CHEMICAL ABSTRACTS, vol. 71, no.23, December 8, 1969 Columbus, Ohio, USA SCHMIDT, GUENTHER et al. "Analgesic 5, 11-dihydro-6H-pyrido (2,3-b)(1,4)benzodiazepin-6-ones." & S. African 68 05,942

CHEMICAL ABSTRACTS, vol. 106, no. 11, march 16, 1987 Columbus, Ohio, USA BORELL BILBAO, JOSE IGNACIO et al. "Process for the preparation of pirenzepine." & Span. ES 548.906

(74) Representative: **Laudien, Dieter, Dr. et al**
**Boehringer Ingelheim GmbH**
**Abteilung Patente**
**D-55216 Ingelheim (DE)**

## Description

In the literature are already described 5,11-dihydro-6H-pyrido[2,3-b][1,4]benzodiazepin-6-ones showing valuable properties, for example antiphlogistic, analgesic, antipyretic, sedative and anticonvulsive activities (see FR-A-1,542,160 and Chemical Abstracts 71, 112 944z (1969)), anti-ulcer activities (see DE-A-1,966,815), an inhibition effect on stress-induced ulceration (see Chemical Abstracts 90, 137 875s (1979)), an inhibition effect on the secretion and formation of ulcus, an anti-tussive and antiemetic activity (see Chemical Abstracts 73, 77292m (1970)), and antihistaminic, antidepressive and antiulcer activities (see Chemical Abstracts 82, 171 104j (1975)). In Chemical Abstract 106, 84661j (1987) is described only a new process for the preparation of pirenzepine.

It was now found that known and novel 5,11-dihydro-6H-pyrido[2,3-b][1,4]benzodiazepin-6-ones and -6-thiones are useful in the prevention or treatment of AIDS.

The human disease, Acquired Immune Deficiency Syndrome (AIDS), is caused by the Human Immunodeficiency Virus (HIV), particularly the strain known as HIV-1.

Like other viruses, HIV-1 cannot replicate without commandering the biosynthetic apparatus of the host cell it infects. It causes this apparatus to produce the structural proteins which make up the viral progeny. These proteins are coded for by the genetic material contained within the infecting virus particle, or virion. Being a retrovirus, however, the genetic material of HIV is RNA, not DNA as in the host cell's genome. Accordingly, the viral RNA must first be converted into DNA, and then integrated into the host cell's genome, in order for the host cell to produce the required viral proteins.

The conversion of the RNA to DNA is accomplished through the use of the enzyme reverse transcriptase (RT), which is included within the infecting virion along with the RNA. Reverse transcriptase has three enzymatic functions; it acts as an RNA-dependent DNA polymerase, as a ribonuclease, and as a DNA-dependent DNA polymerase. Acting first as an RNA-dependent DNA polymerase, RT makes a single-stranded DNA copy of the viral RNA. Next, acting as a ribonuclease, RT frees the DNA just produced from the original viral RNA and then destroys the original RNA. Finally, acting as a DNA-dependent DNA polymerase, RT makes a second complementary, DNA strand, using the first DNA strand as a template. The two strands form double-stranded DNA, the form of DNA found in the host cell's genome, which is integrated into the host cell's genome by another enzyme, called an integrase.

Compounds which inhibit the enzymatic functions of HIV-1 reverse transcriptase will inhibit replication of HIV-1 in infected cells. Such compounds are useful in the prevention or treatment of HIV-1 infection in human subjects.

### Summary of the Invention

A first aspect of the invention comprises a method for preventing or treating HIV-1 infection which comprises administering, to a human exposed to or infected by HIV-1, a prophylactically or therapeutically effective amount of certain 5,11-dihydro-6H-pyrido[2,3-b][1,4]benzodiazepin-6-ones and -thiones. Some of these compounds are novel and some are known. All possess inhibitory activity against HIV-1 RT. A second aspect of the invention comprises novel 5,11-dihydro-6H-pyrido[2,3-b][1,4]benzodiazepin-6-thiones. A third aspect of the invention comprises methods for making these novel compounds. A final aspect of the invention comprises pharmaceutical compositions suitable for the prevention or treatment of HIV-1 infection comprising the above mentioned compounds, both novel and known.

### Detailed Description of the Invention

A first aspect of the invention comprises a method for preventing or treating HIV-1 infection which comprises administering to a human, exposed to or infected by HIV-1, a prophylactically or therapeutically effective amount of a 5,11-dihydro-6H-pyrido[2,3-b][1,4]benzodiazepin-6-oneor -thione of the formula

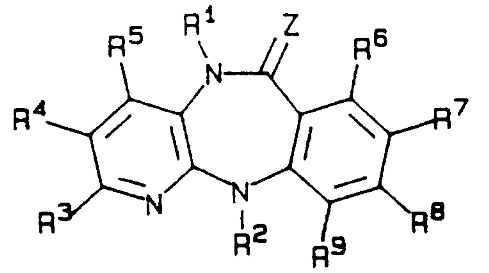

(I)

Z is oxygen or sulfur;

$R^1$ is hydrogen, alkyl or fluoroalkyl of 1 to 4 carbon atoms, cyclopropyl, alkenyl or alkynyl of 3 to 4 carbon atoms, 2-halo-propen-1-yl, arylmethyl (wherein the aryl moiety is phenyl or thienyl, which is either unsubstituted or substituted by methyl, methoxy or halogen), acetyl, or alkoxyalkyl or alkylthioalkyl of 2 to 3 carbon atoms;

$R^2$ is alkyl or fluoroalkyl of 1 to 4 carbon atoms, cycloalkyl of 3 to 5 carbon atoms, alkenyl or alkynyl of 2 to 4 carbon atoms, alkoxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl, thienyl or furanyl, which is either unsubstituted or substituted by alkyl or alkoxy of 1 to 3 carbon atoms, hydroxyl or halogen), phenyl (which is either unsubstituted or substituted by alkyl or alkoxy of 1 to 3 carbon atoms, halogen or hydroxyl) or alkoxycarbonylmethyl wherein the alkoxy moiety contains 1 to 5 carbon atoms;

$R^3$, $R^4$ and $R^5$ are each independently hydrogen or alkyl of 1 to 3 carbon atoms, with the proviso that at least one of these substituents is hydrogen; or,

one of $R^3$, $R^4$ and $R^5$ is butyl, alkanoyl of 2 to 4 carbon atoms, alkoxycarbonyl or 2 to 4 carbon atoms, hydroxyalkyl of 1 to 4 carbon atoms, alkoxycarbonylalkyl wherein the alkoxy and alkyl moieties each contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido, mono- or di-alkylaminoalkyl wherein each alkyl moiety contains 1 to 2 carbon atoms, with the proviso that the remaining two substituents are hydrogen or methyl; or,

when Z is oxygen, one of $R^3$, $R^4$ and $R^5$ is alkylsulfinyl or alkylsulfonyl of 1 to 3 carbon atoms, with the proviso that the remaining two substituents are hydrogen or methyl; and

$R^6$, $R^7$, $R^8$ and $R^9$ are each hydrogen; or

one of $R^6$, $R^7$, $R^8$ and $R^9$ is alkyl of 1 to 4 carbon atoms, alkanoyl of 2 to 4 carbon atoms, alkoxycarbonyl of 2 to 4 carbon atoms, hydroxyalkyl of 1 to 4 carbon atoms, alkoxycarbonylalkyl wherein the alkoxy and alkyl moieties each contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido, mono- or di-alkylaminoalkyl where in each alkyl moiety contains 1 to 2 carbon atoms, and the remaining three substituents are hydrogen or two of the remaining three substituents are hydrogen and one is methyl, ethyl or halogen.

In a subgeneric aspect, the invention comprises the above-described method wherein, in the compound of formula I,

Z is oxygen or sulfur;

$R^1$ is hydrogen, alkyl of 1 to 3 carbon atoms, allyl, propargyl,
2-halo-propen-1-yl, methoxymethyl or methylthiomethyl;

$R^2$ is alkyl 1 to 4 carbon atoms, cycloalkyl of 3 to 4 carbon atoms, alkenyl or alkynyl of 2 to 4 carbon atoms, alkoxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl, methoxy or halogen) or alkoxycarbonylmethyl wherein the alkoxy moiety contains 1 to 4 carbon atoms;

$R^3$, $R^4$ and $R^5$ are each independently hydrogen or methyl, with the proviso that at least one of these substituents is hydrogen, or $R^5$ is ethyl, propyl or butyl with the remaining two substituents being hydrogen;

$R^6$ is chosen from hydrogen, methyl and ethyl but can only have either of the latter two identities when $R^7$ is hydrogen or methyl;

$R^7$ and $R^8$ are chosen from hydrogen, methyl or halogen, or
$R^7$ is alkyl of 1 to 2 carbon atoms, acetyl, hydroxyalkyl of 1 to 2 carbon atoms, alkoxycarbonyl of 2 to 3 carbon atoms, alkoxycarbonylalkyl, wherein the alkoxy and alkyl moieties each contain 1 to 2 carbon atoms, halogen, trifluoromethyl, hydroxyl, alkoxy of 1 to 2 carbon atoms, alkylthio of 1 to 2 carbon atoms, acetyloxy, alkanoylamino of 1 to 2 carbon atoms or cyano, and $R^8$ is hydrogen; or
$R^8$ is alkyl of 1 to 2 carbon atoms, acetyl, hydroxyalkyl of 1 to 2 carbon atoms, alkoxycarbonyl of 2 to 3 carbon atoms, alkoxycarbonylalkyl, wherein the alkoxy and alkyl moieties each contain 1 to 2 carbon atoms, halogen, trifluoromethyl, hydroxyl, alkoxy of 1 to 2 carbon atoms, alkylthio of 1 to 2 carbon atoms, alkanoylamino of 1 to 2 carbon atoms or cyano, and $R^7$ is hydrogen; and
$R^9$ is chosen from hydrogen and methyl but can only have the latter identity when $R^8$ is hydrogen or methyl.

In a further subgeneric aspect, the invention comprises the above described methods wherein in the compound of firmula I
Z is oxygen or sulfur;
$R^1$ is hydrogen, 2-halo-2-propen-1-yl, or alkyl of 1 to 3 carbon atoms;
$R^2$ is alkyl of 1 to 4 carbon atoms or cycloalkyl of 3 to 4 carbon atoms; and,
$R^3$ through $R^9$ are each hydrogen.

Compounds of formula I may, if desired, be converted into their non-toxic, pharmaceutically acceptable acid addition salts by conventional methods; for example, by dissolving a compound of formula I in a suitable solvent and acidifying the solution with one or more molar equivalents of the desired acid. The invention also comprises the use of such salts. Salt formation at any of $R^3$ through or $R^9$, when these ale basic amines, is preferred.

Examples of inorganic and organic acids which may form non-toxic, pharmaceutically acceptable acid addition salts with a compound of formula I are the following: hydrochloric acid, hydrobromic acid, sulfuric acid, phosphoric acid, nitric acid, tartaric acid, citric acid, methanesulfonic acid and the like. Compounds of formula I usually form acid addition salts with one molar equivalent of the acid.

The above described compounds of formula I inhibit HIV-1 reverse transcriptase and thereby inhibit HIV-1 replication, making them useful in the method which constitutes the first aspect of the invention.

In carrying out this method, the compounds formula I may be administered in single or divided doses by the oral, parenteral or topical routes. A suitable oral dosage for such compounds would be in the range of about 10 to 500 mg per day. In parenteral formulations, a suitable dosage unit may contain from 1 to 50 mg of said compounds, whereas for topical administration, formulations containing 0.01 to 1% active ingredient are preferred. It should be understood, however, that the dosage administration from patient to patient will vary and the dosage for any particular patient will depend upon the clinician's judgement, who will use as criteria for fixing a proper dosage the size and condition of the patient as well as the patient's response to the drug.

When such compounds are to be administered by the oral route, they may be administered as medicaments in the form of pharmaceutical preparations which contain them in association with a compatible pharmaceutical carrier material. Such carrier material can be an inert organic or inorganic carrier material suitable for oral administration. Examples of such carrier materials are water, gelatin, talc, starch, magnesium stearate, gum arabic, vegetable oils, polyalkyleneglycols, petroleum jelly and the like. The pharmaceutical preparations can be prepared in a conventional manner and finished dosage forms can be solid dosage forms, for example, tablets, dragees, capsules, and the like, or liquid dosage forms, for example, solutions, suspensions, emulsions and the like. The pharmaceutical preparations may be subjected to conventional pharmaceutical operations such as sterilization. Further, the pharmaceutical preparations may contain conventional adjuvants such as preservatives, stabilizers, emulsifiers, flavor-improvers, wetting agents, buffers, salts for varying the osmotic pressure and the like. Solid carrier material which can be used include, for example, starch, lactose, mannitol, methyl cellulose, microcrystalline cellulose, talc, silica, dibasic calcium phosphate, and high molecular weight polymers, such as polyethylene glycol.

For parenteral use, it is preferred to administer such compounds in an aqueous or non-aqueous solution, suspension or emulsion in a pharmaceutically acceptable oil or a mixture of liquids, which may contain bacteriostatic agents, antioxidants, preservatives, buffers or other solutes to render the solution isotonic with the blood, thickening agents, suspending agents or other pharmaceutically acceptable additives. Additives of this type include, for example, tartrate, citrate and acetate buffers, ethanol, propylene glycol, polyethylene glycol, complex formers (such as EDTA), antioxidants (such as sodium bisulfite, sodium metabisulfite, and ascorbic acid), high molecular weight polymers (such as liquid polyethylene oxides) for viscosity regulation and polyethylene derivatives of sorbitol anhydrides. Preservatives may also be added if necessary, such as benzoic acid, methyl or propyl paraben, benzalkonium chloride and other

quaternary ammonium compounds.

The compounds can also be administered as solutions for nasal applications which may contain, in addition to the compounds, suitable buffers, tonicity adjusters, microbial preservatives, antioxidants and viscosity-increasing agents in an aqueous vehicle. Examples of agents used to increase viscosity are polyvinyl alcohol, cellulose derivatives, polyvinyl-pyrrolidone, polysorbates or glycerin. Microbial preservatives added may include benzalkonium chloride, thimerosal, chlorobutanol or phenylethyl alcohol.

Additionally, such compounds can be administered by suppository.

In its composition of matter aspect, the invention comprises novel compounds of formula I, wherein $R^1$ through $R^9$ are as set forth above and Z is sulfur, and pharmaceutically acceptable acid addition salts thereof.

The invention also comprises the following novel compounds of formula I, wherein Z is oxygen, as well as the pharmaceutically acceptable acid addition salts thereof:

a) 5-(2-Chloro-2-propenyl)-11-ethyl-5,11-dihydro-6H-pyrido[2,3-b][1,4]-benzodiazepin-6-one;
b) 2,5,8,11-Tetramethyl-5,11-dihydro-6H-pyrido[2,3-b][1,4]-benzodiazepin-6-one;
c) 5,11-Diechyl-10-methyl-5,11-dihydro-6H-pyrido[2,3-b][1,4]-benzodiazepin-6-one;
d) 5,8-Dimethyl-11-ethyl-5,11-dihydro-6H-pyrido[2,3-b][1,4]-benzodiazepin-6-one;
e) 2,5,10,11-Tetramethyl-5,11-dihydro-6H-pyrido[2,3-b][1,4]-benzodiazepin-6-one;
f) [11-[5,11-dihydro-6-oxo-6H-pyrido[2,3-b][1,4]benzodiazepin-11-yl]]acetic acid, tert-butylester;
g) 2-Chloro-5-ethyl-11-methyl-5,11-dihydro-6H-pyrido[2,3-b][1,4]-benzodiazepin-6-one; and,
h) 5,8,11-Trimethyl-5,11-dihydro-6H-pyrido[2,3-b][1,4]-benzodiazepin-6-one.

Compounds of formula I, wherein Z is oxygen can be prepared according to the following general methods A, B and C.

Method A

In this method, a compound of the formula

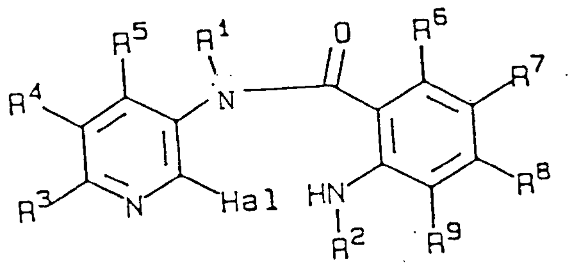

(II)

wherein Hal is a halogen atom and $R^1$ through $R^9$ are as set forth above, is cyclized. The cyclisation may be effected by heating the compound of formula II to elevated temperatures, preferably above 150 °C, advantageously in the presence of a high-boiling point inert solvent, such as trichlorobenzene, paraffin oil or sulfolane, in the presence of a basic catalyst, such as potash, or copper powder, but preferably in the presence of an acid-catalyst such as hydrogen chloride or concentrated sulfuric acid. Upon heating the compound of formula II, the evolution of hydrogen halide begins at temperatures above 150 °C, which ceases after heating for several hours. Thereafter, the high-boiling-point solvent which may be present, is distilled off *in vacuo*, and the desired end product of formula I crystallizes when cooling. The raw product is then purified by conventional methods, such as recrystallization from an inert solvent.

Method B

In this method, an isatoic acid of the formula

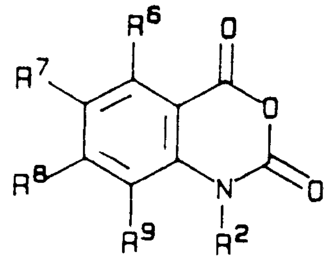

(III)

wherein $R^2$ and $R^6$ through $R^9$ are as set forth above, is reacted with a 2-halo-3-amino pyridine of the formula

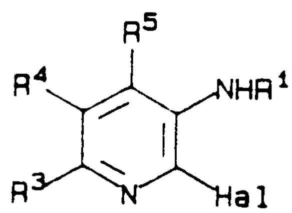

(IV)

wherein $R^1$ and $R^3$ through $R^5$ are as set forth above and Hal is a halogen atom. The reaction is carried out at a temperature above 150 °C. This method makes it possible to prepare a compound of formula I with good yields by means of a continuous reaction procedure. The reaction is carried out either without a solvent medium or in the presence of a high-boiling point inert solvent, such as trichlorobenzene, tetraethyleneglycol, tetrahydronaphthalene, sulfolane or the like and preferably in the presence of catalytic amounts of a mineral acid such as hydrogen chloride or concentrated sulfuric acid. Upon heating the reaction mixture, carbon dioxide is initially evolved at temperatures between 120 and 150 °C and a compound of formula II above is formed as an intermediate. However, this intermediate need not be isolated; instead, the intermediate is cyclized in situ, accompanied by evolution of hydrogen halide, by heating the reaction mixture containing it to a temperature above 150 °C, preferably to between 180 and 250 °C. Of course, the reaction mixture of the compounds of formulas III and IV may also be heated right away to the temperature required for cyclisation.

Method C

In those instances where methods A or B yield a compound of formula I wherein $R^1$ is hydrogen, such compound may, if desired, be converted, using conventional methods, into a compound of formula I wherein $R^1$ is other than hydrogen. To accomplish this, a compound of formula I, wherein $R^1$ is hydrogen, is first reacted with an alkali metal hydroxide, alkali metal alcoholate, alkali metal amide or alkali metal hydride to form a 5-alkali metal compound as an intermediate. The 5-alkali metal compound thus obtained is then reacted with a compound of the formula $R^1Y$, wherein $R^1$ has the same definitions as set forth above, except for hydrogen, and Y is a leaving group such as chlorine, bromine, iodine, the anion of a reactive ester or the radical of a sulfuric acid ester or of an aliphatic or aromatic sulfonic acid ester.

The preparation of the starting compounds of formula II is described in US-Patents 3,539,554 and 3,406,168. The starting compounds of formulae III and IV are all known or can be produced in accordance with methods described in the literature.

As stated before, the compounds provided by the invention inhibit the enzymatic activity of HIV-1 RT. Based upon testing of these compounds, as described below, it is known that they inhibit the RNA-dependent DNA polymerase activity of HIV RT. Based upon other testing, not described herein, it is believed that they also inhibit the DNA-dependent DNA polymerase activity of HIV RT.

Utilizing the Reverse Transcriptase (RT) Assay described below, compounds can be tested for their ability to inhibit the RNA-dependent DNA polymerase activity of HIV RT. Certain specific compounds, described in the Examples which appear below, were so tested. The results of this testing appear in Table I, below.

REVERSE TRANSCRIPTASE (RT) ASSAY

Assay Theory:

Among the enzymes for which Human Immunodeficiency Virus (HIV-1) encodes is a reverse transcriptase (1), so-named because it transcribes a DNA copy from an RNA template. This activity can be quantitatively measured in a cell-free enzyme assay which has been previously described (2), and is based upon the observation that reverse transcriptase is able to use a synthetic template [poly r(C) primed with oligo d(G)] to transcribe a radio-labelled, acid-precipitable DNA strand utilizing $^3$H-dGTP as a substrate.

Materials:

a) Preparation of the enzyme

Reverse transcriptase enzyme from the LAV strain of Human Immunodeficiency Virus (HIV-1) (1) was isolated from the bacterial strain JM109 (3) expressing the DNA clone pBRTprt1+ (2) which is under the control of the lac promotor in the expression vector pIBI21 (4). An overnight culture grown in 2XYT medium (37°C, 225 rpm) (5) supplemented with 100 µg/ml ampicillin for positive selection is inoculated at a 1:40 dilution into M9 medium supplemented with 10µg/ml thiamine, 0.5% casamino acids, and 50 µg/ml ampicillin (5). The culture is incubated (37°C, 225 rpm) until it reaches an OD540 of 0.3-0.4. At that time the repressor inhibitor IPTG (isopropyl b-D-thiogalactopyranoside) is added to 0.5mM and incubated for 2 additional hours. Bacteria are pelleted, resuspended in a 50mM Tris, 0.6mM EDTA, 0.375M NaCl buffer and digested by the addition of lysozyme (1mg/ml) for 30 minutes on ice. The cells are lysed by the addition to 0.2% NP-40 and brought to 1M NaCl.

After removal of the insoluble debris by centrifugation, the protein is precipitated by the addition of 3 volumes of saturated aqueous ammonium sulfate. The enzyme is pelleted, resuspended in RT buffer (50mM Tris pH 7.5, 1mM EDTA, 5mM DTT, 0.1% NP-40, 0.1M NaCl, and 50% glycerol) and stored at -70°C for further use.

b) Composition of 2X concentrated stock reaction mixture

| Stock Reagent | 2X Mix Concentration |
|---|---|
| 1M Tris pH 7.4 | 100mM |
| 1M Dithiothrietol | 40mM |
| 1M NaCl | 120mM |
| 1% Nonidet P-40 | 0.1% |
| 1M MgCl | 4mM |
| [poly r(C) /oligo d(G)](5:1) | 2µg/ml |
| $^3$H-dGTP (81µM) | 0.6µM |

Assay Procedure:

The 2X concentrated stock reaction mixture is aliquoted and stored at -20°C. The mixture is stable and thawed for use in each assay. This enzyme assay has been adapted to a 96 well microtiter plate system, and has been previously described (6). Tris buffer (50 mM, pH 7.4), vehicle (solvent diluted to match the compound dilution), or compounds in vehicle are dispensed into 96-well miciotiter plates (10µl/well; 3 wells/compound). The HIV RT enzyme is thawed, diluted in 50mM Tris pH 7.4 so that fifteen µl of diluted enzyme contain 0.001 Unit (one unit is that amount of enzyme to transform 1 micromole of substrate per minute at 25°C), and 15µl are dispensed per well. Twenty µl of 0.12-0.5M EDTA are added to the first three wells of the microtiter plate. EDTA chelates the $Mg^{++}$ present and prevents reverse transcription. This group serves as background polymerization which is subtracted from all other groups. Twenty-five µl of the 2X reaction mixture are added to all wells and the assay is allowed to incubate at room temperature for 60 minutes. The assay is terminated by precipitating the DNA in each well with 50µl of trichloracetic acid

(TCA) (10% w/v) in sodium pyrophosphate (1% w/v). The microtiter plate is incubated for 15 minutes at 4°C and the precipitate is fixed onto #30 glass fiber paper (Schleicher & Schuell) using a Skatron semi-automatic harvester. The filters are then washed with additional TCA (5%) containing sodium pyrophosphate (1%), rinsed with aqueous ethanol (70%), dried, and transferred to scintillation vials (6). Each vial receives 2 mls of scintillation cocktail and is counted in a Beckman beta counter.

The calculation for percent inhibition is as follows:

$$\% \text{ inhibition} = \frac{\text{CPM Mean Test Value} - \text{CPM Mean Control Value X100}}{\text{CPM Mean Control Value}}$$

References:

1. Benn, S., et al., SCIENCE 230:949, 1985
2. Farmerie, W.G. et. al., SCIENCE 236:305, 1987
3. Yanisch-Perron, C., Viera, J., and Messing, J., GENE 33:103, 1985
4. International Biotechnologies, Inc., New Haven, CT 06535
5. Maniatis, T, Fritsch, E.F., and J. Sambrook, eds. MOLECULAR CLONING: A LABORATORY MANUAL, Cold Spring Harbor Laboratory, 1982
6. Spira, T., et. al. J. Clinical Microbiology, 25:97, 1987.

In order to confirm that compounds which are active in the RT Assay also have the ability to inhibit HIV replication in a living system, compounds according to the invention were also tested in the Human T-Cell Culture Assay described below. The results of this testing appear in Table I.

HUMAN T CELL CULTURE ASSAY

Assay Theory: Formation of syncytia is a feature of in vitro cultures of CD4+ T-cells infected with HIV-1. In this assay, T-cells are treated with a putative replication inhibiting compound and then infected with HIV-1. After incubation the culture is checked for the formation of syncytia. The absence or reduction is the number of syncytia is used as a measure of the test compound's ability to inhibit HIV replication.

Assay Method: The target cells, designated c8166, are a subclone of human lymphoma cells of T-cell origin and are established at an initial density of $5x10^4$ per 100 µl in RPMI 1640 (+ 10% fetal bovine serum) culture medium in 96 well flat bottom plates. A selected amount of test compound, dissolved in DMSO is included. After 24 hours, 50-100 $TCID_{50's}$ (the dose that results in induced effect in 50% of test cultures) of the HTLV-IIIB strain of HIV-1 (2) are innoculated into each culture. Control cultures receive compound or virus only. Four days after virus challenge, cultures are visually examined for the frequency and distribution of virus-induced giant cell syncytia. The percent inhibition by the test compound is determined by comparison with control values. Confirmation of the presence or absence of virus replication is accomplished by harvesting the cell free culture fluids from all experimental groups to determine the presence or absence of infectious progeny through the induction of syncytia formation in secondary human T-cell cultures after 3 days.

References:

(1) M. Somasundaran and H.L. Robinson, Science 242, 1554 (1998)
(2) G.M. Shaw, R.H. Hahn, S.K. Arya, J.E. Groopman, R.C. Gallo and F. Wong-Staal, Science 226, 1165 (1984)

In order to assess the specificity of the enzyme inhibitory activity of the compounds provided by the invention, a few were tested, using known per se assay methods, for their ability to inhibit Feline Leukemia Virus-derived reverse transcriptase and Calf Thymus-derived DNA alpha-polymerase. None of the compounds so tested was observed to possess any inhibitory activity against these enzymes. These results indicate that the enzyme inhibitory activity of the compounds provided by the invention is directed rather specifically against HIV RT.

In order to roughly assess the cytotoxicity of the compounds provided by the invention, several such compounds were tested in the MTT Cellular Cytotoxicity Assay described below. The results of this testing are reported in Table I, below. Compounds having a relatively high $EC_{50}$ are preferred.

MTT ASSAY FOR CELLULAR CYTOTOXICITY

Assay Theory:

The MTT [3-(4,5-dimethylthiazol-2-yl)-2,5-diphenyl tetrazolium bromide) assay is based on cleavage of tetrazolium bromide by metabolically active cells, resulting in a highly quantitative blue color. This assay has been previously described (1) but his been optimized for the purposes of the testing reported herein.

Assay Method:

The H9 cell line (2), an established human lymphoma suspension cell line grown in RPMI 1640 supplemented with 10% fetal bovine serum is used as the target cell line in the assay. Cells (100µl) are plated in microtest plate wells at a concentration of $10^5$ cells per ml in the presence of varying concentrations of inhibitor. The cells are incubated at 37°C in a humidified $CO_2$ incubator. Five days later, 20µl of MTT (5mg/ml in RPMI 1640, sonicated, 0.2 micron filtered, and stored at 4°C) is added to each well. After 4 hours additional incubation at 37°C, 60µl of Triton-X is added to each well and thoroughly mixed to aid the solubilization of the crystals. Absolute ethanol (5µl) is added to each well and the resulting mixture is incubated for 30 minutes at 60°C and immediately read on a plate reader (Dynatech) at a wavelength of 570nm.

Data from this assay are used to generate a nonlinear regression analysis which yields an $EC_{50}$, the highest non-toxic concentration.

References:

1. Mosmann, Tim, J. Immunol. Methods, 65:55, 1983.
2. Jacobs, J.P., J. Natl. Cancer Inst., 34:231, 1965.

TABLE I

| Compound of Example | RT Inhibition (% @ 10µg/ml) | T-Cell Culture Assay(%inhibition) | Cytotoxity Assay ($EC_{50}$) |
|---|---|---|---|
| 1 | 41 | NT | NT |
| 2 | 50 | " | " |
| 3 | 87 | " | " |
| 4 | 91 | 100%@19µM | 40µM |
| 5 | 84 | " | NT |
| 6 | 86 | " | " |
| 7 | 43 | " | " |
| 8 | 40 | " | " |
| 9a | 17 | " | " |
| 9b | 82 | " | " |
| 9c | 55 | " | " |
| 9d | 24 | " | " |
| 9e | 38 | " | " |
| 9f | 94 | " | " |
| 9g | 87 | 100%@75µM | 100µM |
| 9h | 70 | NT | NT |
| Note: NT = not tested | | | |

The following examples further illustrate the present invention and will enable others skilled in the art to understand the invention more completely.

**EXAMPLE 1**

5-(2-Chloro-2-propenyl)-11-ethyl-5,11-dihydro-6H-pyrido [2,3-b][1,4]benzodiazepin-6-one

9.7g (0.034 mol) of 5-(2-chloro-2-propenyl)-11-ethyl-5-,11-dihydro-6H-pyrido[2,3-b][1,4]benzodiazepin-6-one, m. p. 157-160 ° C, were suspended in 100 ml of anhydrous dimethylformamide. The mixture was thoroughly purged with dry nitrogen and stirred rapidly while 1.8g (0.0375 mol) of a 50% dispersion of sodium hydride in mineral oil was slowly added portionwise through an inlet protected by a calcium chloride drying tube. Addition of the first portion sodium hydride resulted in an immediate exothermic reaction, and the temperature of the mixture rose rapidly to about 60 ° C. After addition of the sodium hydride was complete (30-35 minutes), the reaction mixture was cooled to room temperature, and 7.8g (0.05 mol) of ethyl iodide were added dropwise. Thereafter, the flask was placed in an oil bath heated to 100 ° C, and the reaction mixture was stirred and heated for 2 hours, during which time a slow stream of dry nitrogen was passed through the apparatus. After this time no starting material was detected by thin-layer-chromatography.

The reaction mixture was cooled to room temperature and, in order to destroy possibly still present sodium hydride, 1 ml of methanol was added. The reaction mixture was evaporated in vacuo, the residue was distributed between 100 ml or ether and 100 ml of water, the organic layer was separated and the aqueous phase was exhaustively extracted with ether. The combined organic layers were dried over sodium sulfate and, after the inorganic salts had been removed by filtration under reduced pressure, the filtrate was evaporated to dryness in vacuo. The highly viscous reddish oil thus obtained was further purified by column chromatography on silica gel (0.2-0.5 mm) using chloroform/ethyl acetate 4/1 (v/v) as an eluent. The crystalline raw material obtained by evaporation of suitable fractions was recrystallized from cyclohexane and from diethyl ether, and yielded 5.9 g (55% of theory) of colorless crystals, having a m.p. of 80-82 ° C.

**EXAMPLE 2**

2,5,8,11-Tetramethyl-5,11-dihydro-6H-pyrido[2,3-b][1,4]benzodiazepin-6-one

Using a procedure analogous to that described in Example 1, 5,11-dihydro-2,5,8,11-tetramethyl-5,11-dihydro-6H-pyrido[2,3-b][1,4]benzodiazepin-6-one m.p. 139-141 ° C (recrystallized from ligroin, b.p. 100-140 ° C) was prepared from 2,5,8,11-tetramethyl-5,11-dihydro-6H-pyrido[2,3-b][1,4]benzodiazepin-6-one, m.p. 204-206 ° C, and methyl iodide. The yield was 78% of theory.

**EXAMPLE 3**

5,11-Diethyl-10-methyl-5,11-dihydro-6H-pyrido[2,3-b][1,4]benzodiazepin-6-one

Using a procedure analogous to that described in Example 1. 5,11-dihydro-6H-pyrido[2,3-b][1,4]-benzodiazepin-6-one, m.p. 143-144 ° C (recrystallized from ligroin, b.p. 100-140- ° C) was prepared from 5-ethyl-10-methyl-5,11-dihydro-6H-pyrido[2,3-b][1,4]benzodiazepin-6-one, m.p. 168-170 ° C, and ethyl iodide. The yield was 54% of theory.

**EXAMPLE 4**

5,8-Dimethy-11-ethyl-5,11-dihydro-6H-pyrido[2,3-b][1,4]benzodiazepin-6-one

Using a procedure analogous to that described in Example 1, 2,5,10,11-tetramethyl-5,11-dihydro-6H-pyrido[2,3-b][1,4]benzodiazepin-6-one, m.p. 131-132 ° C (recrystallized from ligroin, b.p. 100-140 ° C), was prepared from 2,5,10-trimethyl-5,11-dihydro-6H-pyrido-[2,3-b][1,4]benzodiazepin-6-one, m.p. 140-142 ° C, and methyl iodide. The yield was 28% of theory.

**EXAMPLE 5**

2,5,10,11-Tetramethyl-5,11-dihydro-6H-pyrido[2,3-b][1,4]benzodiazepin-6-one

Using a procedure analogous to that described in Example 1, 2,5,10,11-tetramethyl-5,11-dihydro-6H-pyrido[2,3-b][1,4]benzodiazepin-6-one, m.p. 131-132 ° C (recrystallized from ligroin b.p. 100-140 ° C), was

prepared from 2,5,10-trimethyl-5,11-dihydro-6H-pyrido[2,3-b][1,4]benzodiazepin-6-one, m.p. 140-142 °C, and methyl iodide. The yield was 28% of theory.

**EXAMPLE 6**

[11-[5-11-Dihydro-6-oxo-6H-pyrido[2,3-b][1,4]benzodiazepin-11-yl]acetic acid tert-butyl ester

A round-bottom flask equipped with a mechanical stirrer, a condenser, a pressure equalizing dropping funnel, a thermometer and a glass joint fitted with a rubber septum was charged with a suspension of 10.5 g (0.05 mol) of 5-11-dihydro-6-oxo-6H-pyrido[2,3-b][1,4]benzodiazepin-6-one in 550 ml of anhydrous tetrahydrofuran. After cooling to 0 °C with an externally applied ice-salt bath, 96 ml (0.149 mol) of a 1.55 molar solution of n-butyl lithium in hexane was added through the septum by injection from a syringe at a temperature below +10 °C. The ice bath was retained for 15 minutes before the mixture was heated to 30-35 °C to complete the metallation. Thereafter, the mixture was cooled to -10 °C, and the solution of 8.0 ml (10.64 g, 0.055 mol) of bromoacetic acid tert-butyl ester in 50 ml of absolute tetrahydrofuran was added dropwise to the stirred mixture. The turbid reaction mixture thus obtained was stirred successively at -10 °C and at room temperature for 2 hours each. The suspension was then stirred into 1L of brine, and the resulting mixture was exhaustively extracted with ethyl acetate. The combined organic layers were washed with water, dried over anhydrous sodium sulfate and filtered. The solvent was evaporated in vacuo, and the residue was purified by column chromatography on silica gel (0.063-0.2 mm) using dichloromethane/ethyl acetate/cyclohexane 1400/400/50 v/v/v as an eluent. Evaporation of suitable fractions yielded a colorless resin that was identified to be 11-[5-11-dihydro-6-oxo-6H-pyrido[2,3-b][1,4]benzodiazepin-11-yl]acetic acid tert-butyl ester. The yield was 5.6 g (34% of theory).

**EXAMPLE 7**

2-Chloro-5-ethyl-11-methyl-5,11-dihydro-6H-pyrido[2,3-b] [1,4]benzodiazepin-6-one

2-Chloro-11-methyl-5,11-dihydro-6H-pyrido[2,3-b][1,4]benzodiazepin-6-one (64.9 g, 0.25 mol) and 20 g (0.285 mol) of potassium methylate were mixed with a mixture consisting of 250 ml of absolute dioxan and 250 ml of tertiary butanol, and the mixture was refluxed for two hours. Thereafter, the reaction solution was allowed to cool, 45 g (0.3 mol) of ethyl iodide were then added dropwise over a period of 45 minutes, and the mixture was again refluxed for two hours. Subsequently, the reaction solution was filtered while still hot, the, filtrate was evaporated in vacuo, and the residue was recrystallized frown cyclohexane. 32.4 g (45% of theory) of 2-chloro-11-methyl-5,11-dihydro-6H-pyrido[2,3-b][1,4]benzodiazepin-6-one, m.p. 162-164 °C, were obtained.

**EXAMPLE 8**

5,8,11-Trimethyl-5,11-dihydro-6H-pyrido[2,3-b][1,4]benzodiazepin-6-one

Using a procedure analogous to that described in Example 1, 5,8,11-trimethyl-5,11-dihydro-6H-pyrido-[2,3-b][1,4]benzodiazepin-6-one, m.p. 147-149 °C (recrystallized from ligroin, b.p. 100-140 °C), was prepared from 5,8-dimethyl-5,11-dihydro-6H-pyrido[2,3-b][1,4]benzodiazepin-6-one, m.p. 176-178 °C, and methyl iodide. The yield was 58% of theory.

**EXAMPLE 9**

The following known compounds were prepared using the procedures set forth in U.S. Patent No. 3,539,544:

a) 11-Methyl-5,11-dihydro-6H-pyrido[2,3-b][1,4]benzodiazepin-6-one;
b) 11-Ethyl-5,11-dihydro-6H-pyrido[2,3-b][1,4]benzodiazepin-6-one;
c) 5,11-dihydro-6H-pyrido[2,3-b][1,4]benzodiazepin-6-one;
d) 5-Ethyl-11-methyl-5,11-dihydro-6H-pyrido[2,3-b][1,4]benzodiazepin-6-one;
e) 5-n-Propyl-11-methyl-5,11-dihydro-6H-pyrido[2,3-b][1,4]benzodiazepin-6-one;
f) 5-Methyl-11-ethyl-5,11-dihydro-6H-pyrido[2,3-b][1,4]benzodiazepin-6-one;
g) 5,11-Diethyl-5,11-dihydro-6H-pyrido[2,3-b][1,4]benzodiazepin-6-one; and
h) 5-(2-Propenyl)-11-ethyl-5,11-dihydro-6H-pyrido[2,3-b][1,4]benzodiazepin-6-one.

EXAMPLE A

| Capsules or Tablets | | | |
|---|---|---|---|
| A-1 | | A-2 | |
| Ingredients | Quantity | Ingredients | Quantity |
| Compound of Example 4 | 50 mg | Example 4 | 50 mg |
| Starch | 160 mg | Dicalcium Phosphate | 160 mg |
| Microcrys, Cellulose | 90 mg | Microcrys. Cellulose | 90 mg |
| Sodium Starch Gluctate | 10 mg | Stearic acid | 5 mg |
| Magnesium Stearate | 2 mg | Sodium Starch Glycolate | 10 mg |
| Fumed colloidal silica | 1 mg | Fumed colloidal silica | 1 mg |

The compound of Example 4 is blended into a powder mixture with the premixed exipient materials as identified above with the exception of the lubricant. The lubricant is then blended in and the resulting blend compressed into tablets or filled into hard gelatin capsules.

EXAMPLE B

| Parenteral Solutions | |
|---|---|
| Ingredients | Quantity |
| Compound of Example 4 | 500mg |
| Ethanol | 25ml |
| Water for injection | q.s. to 100 ml |

Compound of Example 4 is added to the ethanol and mixed until the solution is clear. Water is added and the resulting solution is then filtered into the appropriate vials or ampoules and sterilized by autoclaving.

EXAMPLE C

| Nasal Solutions | |
|---|---|
| Ingredients | Quantity |
| Compound of Example 4 | 500 mg |
| Propylene glycol | 30 ml |
| Benzalkonium chloride | 200 mg |
| EDTA | 200 mg |
| Water | q.s. to 100 ml |

The excipient materials are mixed and thereafter the compound of Example 4 is added and mixing is continued until the solution is clear. The water is added and the resulting solution is then filtered into the appropriate vials or ampoules.

## Claims

**1.** The use of a compound of the formula I

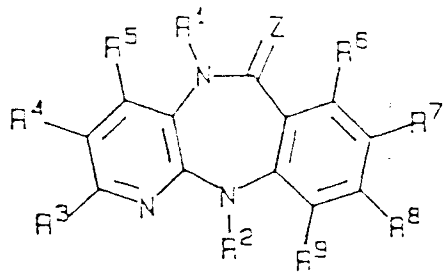

(I)

wherein,
Z is oxygen or sulfur;
$R^1$ is hydrogen, alkyl or fluoroalkyl of 1 to 4 carbon atoms, cyclopropyl, alkenyl or alkynyl of 3 to 4 carbon atoms, 2-halo-propen-1-yl, arylmethyl (wherein the aryl moiety is phenyl or thienyl, which is either unsubstituted or substituted by methyl, methoxy or halogen), acetyl, or alkoxyalkyl or alkylthioalkyl of 2 to 3 carbon atoms;
$R^2$ is alkyl or fluoroalkyl of 1 to 4 carbon atoms, cycloalkyl of 3 to 5 carbon atoms, alkenyl or alkynyl of 2 to 4 carbon atoms, alkoxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl, thienyl or furanyl, which is either unsubstituted or substituted by alkyl or alkoxy of 1 to 3 carbon atoms, hydroxyl or halogen), phenyl (which is either unsubstituted or substituted by alkyl or alkoxy of 1 to 3 carbon atoms, halogen or hydroxyl) or alkoxycarbonylmethyl wherein the alkoxy moiety contains 1 to 5 carbon atoms;
$R^3$, $R^4$ and $R^5$ are each independently hydrogen or alkyl of 1 to 3 carbon atoms, with the proviso that at least one of these substituents is hydrogen; or
one of $R^3$, $R^4$ and $R^5$ is butyl, alkanoyl of 2 to 4 carbon atoms, alkoxycarbonyl of 2 to 4 carbon atoms, hydroxyalkyl of 1 to 4 carbon atoms, alkoxycarbonylalkyl wherein the alkoxy and alkyl moieties each contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido, mono- or di-alkylaminoalkyl wherein each alkyl moiety contains 1 to 2 carbon atoms, with the proviso that the remaining two substituents are hydrogen or methyl; or,
when Z is oxygen, one of $R^3$, $R^4$ and $R^5$ is alkylsulfinyl or alkylsulfonyl of 1 to 3 carbon atoms with the proviso that the remaining two substituents are hydrogen or methyl; and
$R^6$, $R^7$, $R^8$ and $R^9$ are hydrogen; or
one of $R^6$, $R^7$, $R^8$ and $R^9$ is alkyl of 1 to 4 carbon atoms, alkanoyl of 2 to 4 carbon atoms, alkoxycarbonyl of 2 to 4 carbon atoms, hydroxyalkyl of 1 to 4 carbon atoms, alkoxycarbonylalkyl wherein the alkoxy and alkyl moieties each contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido, mono- or di-alkylaminoalkyl wherein each alkyl moiety contains 1 to 2 carbon atoms, and the remaining three substituents are hydrogen or two of the remaining three substituents are hydrogen and one is methyl, ethyl or halogen;
or a pharmaceutically acceptable acid addition salt thereof
in the manufacture of a pharmaceutical composition for the prophylaxis or treatment of a human exposed to or infected by HIV-1.

**2.** The use of claim 1, wherein, in the compound of formula I,
Z is oxygen or sulfur;
$R^1$ is hydrogen, alkyl of 1 to 3 carbon atoms, allyl, propargyl, 2-halo-propen-1-yl, methoxymethyl or methylthiomethyl;

$R^2$ is alkyl of 1 to 4 carbon atoms, cycloalkyl of 3 to 4 carbon atoms, alkenyl or alkynyl of 2 to 4 carbon atoms, alkoxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl or methoxy or halogen) or alkoxycarbonylmethyl wherein the alkoxy moiety contains 1 to 4 carbon atoms;
$R^3$, $R^4$ and $R^5$ are each independently hydrogen or methyl, with the proviso that at least one of these substituents is hydrogen, or $R^5$ is ethyl, propyl or butyl with the remaining two substituents being hydrogen;
$R^6$ is chosen from hydrogen, methyl and ethyl but can only have either of the latter two identities when $R^7$ is hydrogen or methyl;
$R^7$ and $R^8$ are chosen from hydrogen, methyl or halogen, or
$R^7$ is alkyl of 1 to 2 carbon atoms, acetyl, hydroxyalkyl of 1 to 2 carbon atoms, alkoxycarbonyl of 2 to 3 carbon atoms, alkoxycarbonylalkyl, wherein the alkoxy and alkyl moieties each contain 1 to 2 carbon atoms, halogen, trifluoromethyl, hydroxyl, alkoxy of 1 to 2 carbon atoms, alkylthio of 1 to 2 carbon atoms, acetyloxy, alkanoylamino of 1 to 2 carbon atoms or cyano, and $R^8$ is hydrogen; or
$R^8$ is alkyl of 1 to 2 carbon atoms, acetyl, hydroxyalkyl of 1 to 2 carbon atoms, alkoxycarbonyl of 2 to 3 carbon atoms, alkoxycarbonylalkyl, wherein the alkoxy and alkyl moieties each contain 1 to 2 carbon atoms, halogen, trifluoromethyl, hydroxyl, alkoxy of 1 to 2 carbon atoms, alkylthio of 1 to 2 carbon atoms, alkanoylamino of 1 to 2 carbon atoms or cyano, and $R^7$ is hydrogen; and
$R^9$ is chosen from hydrogen and methyl but can only have the latter identity when $R^8$ is hydrogen or methyl.

**3.** The use of claim 1, wherein in the compound of formula I Z is oxygen or sulfur;
$R^1$ is hydrogen, 2-halo-2-propen-1-yl, or alkyl of 1 to 3 carbon atoms;
$R^2$ is alkyl of 1 to 4 carbon atoms or cycloalkyl of 3 to 4 carbon atoms; and
$R^3$ through $R^9$ are each hydrogen.

**4.** A compound selected from the group consisting of the following:
a) 5-(2-Chloro-2-propenyl)-11-ethyl-5,11-dihydro-6H-pyrido[2,3-b][1,4]benzodiazepin-6-one;
b) 2,5,8,11-Tetramethyl-5,11-dihydro-6H-pyrido[2,3-b] [1,4]benzodiazepin-6-one;
c) 5,11-Diethyl-10-methyl-5,11-dihydro-6H-pyrido[2,3-b] [1,4]benzodiazepin-6-one;
d) 5,8-Dimethyl-11-ethyl-5,11-dihydro-6H-pyrido[2,3-b] [1,4]benzodiazepin-6-one;
e) 2,5,10,11-Tetramethyl-5,11-dihydro-6H-pyrido[2,3-b] [1,4]benzodiazepin-6-one;
f) [11-[5,11-Dihydro-6-oxo-6H-pyrido[2,3-b][1,4]benzodiazpin-11-yl]]-acetic acid tert-butylester;
g) 2-Chloro-5-ethyl-11-methyl-5,11-dihydro-6H-pyrido [2,3-b][1,4]benzodiazepin-6-one; and
h) 5,8,11-Trimethyl-5,11-dihydro-6H-pyrido[2,3-b] [1,4]benzodiazepin-6-one;
and pharmaceutically acceptable acid addition salts thereof.

**5.** A method of preparing a compound as defined in claim 4, which comprises carrying out one of the following methods;
A) cyclising a compound of the formula

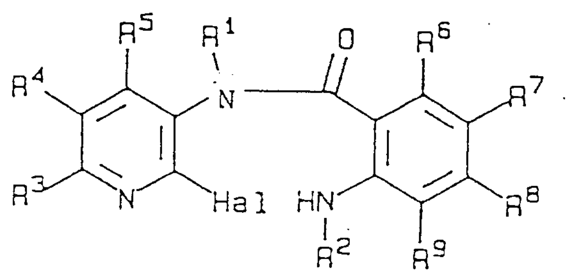

(II)

wherein Hal is a halogen atom and $R^1$ to $R^9$ have the same identities as the corresponding substituents in the resulting compound of claim 4;

B) reacting an isatoic acid of the formula

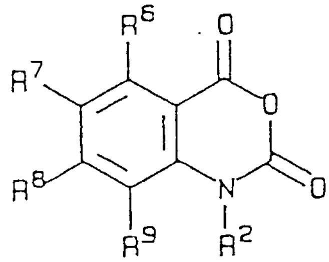

(III)

with a 2-halo-3-aminopyridine of the formula

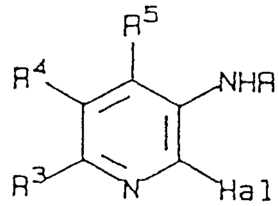

(IV)

wherein $R^2$ to $R^9$ have the same identities as the corresponding substituents in the resulting compound of claim 4, and Hal is a halogen atom; or

C) to produce a compound of claim 4, wherein the substituent at the 5-position is other than hydrogen, reacting a 5-alkali metal derivative of a compound corresponding to the desired compound of claim 4 with a compound of the formula $R^{1'}Y$, wherein $R^{1'}$ corresponds to the 5-substituent of the desired compound of claim 4 and Y is a leaving group,

and thereafter isolating the resulting compound of claim 4 of method A, B or C as such or as a pharmaceutically acceptable acid addition salt.

**6.** A method of preparing a pharmaceutical composition which comprises mixing a compound according to claim 4 with a pharmaceutically acceptable carrier or excepient.

**Patentansprüche**

**1.** Verwendung einer Verbindung der Formel I

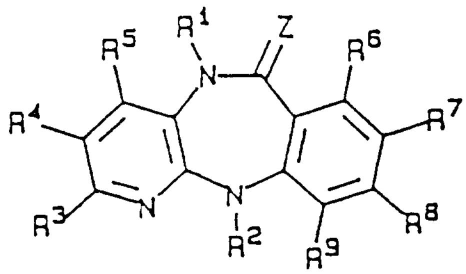

(I)

worin:

Z Sauerstoff oder Schwefel ist;

$R^1$ Wasserstoff, Alkyl oder Fluoralkyl mit 1 bis 4 Kohlenstoffatomen, Cyclopropyl, Alkenyl oder Alkinyl mit 3 bis 4 Kohlenstoffatomen, 2-Halogen-propen-1-yl, Arylmethyl (worin die Arylkomponente Phenyl oder Thienyl ist, das entweder nichtsubstituiert oder mit Methyl, Methoxy oder Halogen substituiert ist), Acetyl oder Alkoxyalkyl oder Alkylthioalkyl mit 2 bis 3 Kohlenstoffatomen ist;

$R^2$ Alkyl oder Fluoralkyl mit 1 bis 4 Kohlenstoffatomen, Cycloalkyl mit 3 bis 5 Kohlenstoffatomen,

Alkenyl oder Alkinyl mit 2 bis 4 Kohlenstoffatomen, Alkoxyalkyl oder Alkylthioalkyl mit 2 bis 3 Kohlenstoffatomen, Alkanoyl mit 2 bis 3 Kohlenstoffatomen, Hydroxyalkyl mit 2 bis 4 Kohlenstoffatomen, Arylmethyl (worin die Arylkomponente Phenyl, Thienyl oder Furanyl ist, das entweder nichtsubstituiert oder mit Alkyl oder Alkoxy mit 1 bis 3 Kohlenstoffatomen, Hydroxyl oder Halogen substituiert ist), Phenyl (das entweder nichtsubstituiert oder mit Alkyl oder Alkoxy mit 1 bis 3 Kohlenstoffatomen, Halogen oder Hydroxyl substituiert ist) oder Alkoxycarbonylmethyl, worin die Alkoxykomponente 1 bis 5 Kohlenstoffatome enthält, ist;

$R^3$, $R^4$ und $R^5$ jeweils unabhängig voneinander Wasserstoff oder Alkyl mit 1 bis 3 Kohlenstoffatomen sind, unter der Bedingung, daß mindestens einer dieser Substituenten Wasserstoff ist; oder

einer der Reste $R^3$, $R^4$ und $R^5$ Butyl, Alkanoyl mit 2 bis 4 Kohlenstoffatomen, Alkoxycarbonyl mit 2 bis 4 Kohlenstoffatomen, Hydroxyalkyl mit 1 bis 4 Kohlenstoffatomen, Alkoxycarbonylalkyl, worin die Alkoxy- und die Alkylkomponente jeweils 1 bis 2 Kohlenstoffatome enthalten, Halogen, Trihalogenmethyl, Hydroxyl, Alkoxy mit 1 bis 3 Kohlenstoffatomen, Alkylthio mit 1 bis 3 Kohlenstoffatomen, Alkanoyloxy mit 2 bis 3 Kohlenstoffatomen, Alkanoylamino mit 1 bis 3 Kohlenstoffatomen, Aminoalkyl mit 1 bis 3 Kohlenstoffatomen, Mono- oder Dialkylamino, worin jede Alkylkomponente 1 bis 2 Kohlenstoffatome enthält, Carboxyalkyl mit 2 bis 3 Kohlenstoffatomen, Cyano, Nitro, Carboxyl, Carbamyl, Amino, Azido, Mono- oder Dialkylaminoalkyl, worin jede Alkylkomponente 1 bis 2 Kohlenstoffatome enthält, ist, unter der Bedingung, daß die übrigen zwei Substituenten Wasserstoff oder Methyl sind; oder

wenn Z Sauerstoff ist, einer der Reste $R^3$, $R^4$ und $R^5$ Alkylsulfinyl oder Alkylsulfonyl mit 1 bis 3 Kohlenstoffatomen ist, unter der Bedingung, daß die übrigen zwei Substituenten Wasserstoff oder Methyl sind; und

$R^6$, $R^7$, $R^8$ und $R^9$ Wasserstoff sind; oder

einer der Reste $R^6$, $R^7$, $R^8$ und $R^9$ Alkyl mit 1 bis 4 Kohlenstoffatomen, Alkanoyl mit 2 bis 4 Kohlenstoffatomen, Alkoxycarbonyl mit 2 bis 4 Kohlenstoffatomen, Hydroxyalkyl mit 1 bis 4 Kohlenstoffatomen, Alkoxycarbonylalkyl, worin die Alkoxy- und die Alkylkomponente jeweils 1 bis 2 Kohlenstoffatome enthalten, Halogen, Trihalogenmethyl, Hydroxyl, Alkoxy mit 1 bis 3 Kohlenstoffatomen, Alkylthio mit 1 bis 3 Kohlenstoffatomen, Alkanoyloxy mit 2 bis 3 Kohlenstoffatomen, Alkanoylamino mit 1 bis 3 Kohlenstoffatomen, Aminoalkyl mit 1 bis 3 Kohlenstoffatomen, Mono- oder Dialkylamino, worin jede Alkylkomponente 1 bis 2 Kohlenstoffatome enthält, Carboxyalkyl mit 2 bis 3 Kohlenstoffatomen, Cyano, Nitro, Carboxyl, Carbamyl, Amino, Azido, Mono- oder Dialkylaminoalkyl, worin jede Alkylkomponente 1 bis 2 Kohlenstoffatome enthält, ist und die übrigen drei Substituenten Wasserstoff sind oder zwei der drei übrigen Substituenten Wasserstoff sind und einer Methyl, Ethyl oder Halogen ist;

oder eines pharmazeutisch brauchbaren Säureadditionssalzes davon

bei der Herstellung einer pharmazeutischen Zusammensetzung zur Prophylaxe oder Behandlung eines Menschen, welcher HIV-1 ausgesetzt oder mit diesem infiziert ist.

**2.** Verwendung gemäß Anspruch 1, worin in der Verbindung der Formel I

Z Sauerstoff oder Schwefel ist;

$R^1$ Wasserstoff, Alkyl mit 1 bis 3 Kohlenstoffatomen, Allyl, Propargyl, 2-Halogen-propen-1-yl, Methoxymethyl oder Methylthiomethyl ist;

$R^2$ Alkyl mit 1 bis 4 Kohlenstoffatomen, Cycloalkyl mit 3 bis 4 Kohlenstoffatomen, Alkenyl oder Alkinyl mit 2 bis 4 Kohlenstoffatomen, Alkoxyalkyl oder Alkylthioalkyl mit 2 bis 3 Kohlenstoffatomen, Alkanoyl mit 2 bis 3 Kohlenstoffatomen, Hydroxyalkyl mit 2 bis 4 Kohlenstoffatomen, Arylmethyl (worin die Arylkomponente Phenyl oder Thienyl ist, das entweder nichtsubstituiert oder mit Methyl oder Methoxy oder Halogen substituiert ist) oder Alkoxycarbonylmethyl, worin die Alkoxykomponente 1 bis 4 Kohlenstoffatome enthält, ist;

$R^3$, $R^4$ und $R^5$ jeweils unabhängig voneinander Wasserstoff oder Methyl sind, unter der Bedingung, daß mindestens einer dieser Substituenten Wasserstoff ist, oder $R^5$ Ethyl, Propyl oder Butyl ist, wobei die übrigen zwei Substituenten Wasserstoff sind;

$R^6$ unter Wasserstoff, Methyl und Ethyl gewählt wird, jedoch nur eine der letzten zwei Identitäten besitzen kann, wenn $R^7$ Wasserstoff oder Methyl ist;

$R^7$ und $R^8$ unter Wasserstoff, Methyl oder Halogen gewählt werden; oder

$R^7$ Alkyl mit 1 bis 2 Kohlenstoffatomen, Acetyl, Hydroxyalkyl mit 1 bis 2 Kohlenstoffatomen, Alkoxycarbonyl mit 2 bis 3 Kohlenstoffatomen, Alkoxycarbonylalkyl, worin die Alkoxy- und die Alkylkomponente jeweils 1 bis 2 Kohlenstoffatome enthalten, Halogen, Trifluormethyl, Hydroxyl, Alkoxy mit 1 bis 2 Kohlenstoffatomen, Alkylthio mit 1 bis 2 Kohlenstoffatomen, Acetyloxy, Alkanoylamino mit 1 bis 2 Kohlenstoffatomen oder Cyano ist und $R^8$ Wasserstoff ist; oder

$R^8$ Alkyl mit 1 bis 2 Kohlenstoffatomen, Acetyl, Hydroxyalkyl mit 1 bis 2 Kohlenstoffatomen, Alkoxycarbonyl mit 2 bis 3 Kohlenstoffatomen, Alkoxycarbonylalkyl, worin die Alkoxy- und die Alkylkomponente jeweils 1 bis 2 Kohlenstoffatome enthalten, Halogen, Trifluormethyl, Hydroxyl, Alkoxy mit 1 bis 2 Kohlenstoffatomen, Alkylthio mit 1 bis 2 Kohlenstoffatomen, Alkanoylamino mit 1 bis 2 Kohlenstoffatomen oder Cyano ist und $R^7$ Wasserstoff ist; und
$R^9$ unter Wasserstoff und Methyl gewählt wird, jedoch nur die letzte Identität besitzen kann, wenn $R^8$ Wasserstoff oder Methyl ist.

**3.** Verwendung gemäß Anspruch 1, worin in der Verbindung der Formel I
Z Sauerstoff oder Schwefel ist;
$R^1$ Wasserstoff, 2-Halogen-2-propen-1-yl oder Alkyl mit 1 bis 3 Kohlenstoffatomen ist;
$R^2$ Alkyl mit 1 bis 4 Kohlenstoffatomen oder Cycloalkyl mit 3 bis 4 Kohlenstoffatomen ist; und
$R^3$ bis $R^9$ jeweils Wasserstoff sind.

**4.** Verbindung, gewählt aus der Gruppe bestehend aus:
a) 5-(2-Chlor-2-propenyl)-11-ethyl-5,11-dihydro-6H-pyrido[2,3-b][1,4]benzodiazepin-6-on;
b) 2,5,8,11-Tetramethyl-5,11-dihydro-6H-pyrido[2,3-b][1,4]benzodiazepin-6-on;
c) 5,11-Diethyl-10-methyl-5,11-dihydro-6H-pyrido[2,3-b][1,4]benzodiazepin-6-on;
d) 5,8-Dimethyl-11-ethyl-5,11-dihydro-6H-pyrido[2,3-b][1,4]benzodiazepin-6-on;
e) 2,5,10,11-Tetramethyl-5,11-dihydro-6H-pyrido[2,3-b][1,4]benzodiazepin-6-on;
f) [11-[5,11-Dihydro-6-oxo-6H-pyrido[2,3-b][1,4]benzodiazepin-11-yl]]-essigsäure-*tert*-butylester;
g) 2-Chlor-5-ethyl-11-methyl-5,11-dihydro-6H-pyrido[2,3-b][1,4]benzodiazepin-6-on; und
h) 5,8,11-Trimethyl-5,11-dihydro-6H-pyrido[2,3-b][1,4]benzodiazepin-6-on;
und pharmazeutisch brauchbaren Säureadditionssalzen davon.

**5.** Verfahren zur Herstellung einer Verbindung, wie sie in Anspruch 4 definiert ist, das die Durchführung einer der folgenden Verfahren umfaßt;
A) die Cyclisierung einer Verbindung der Formel

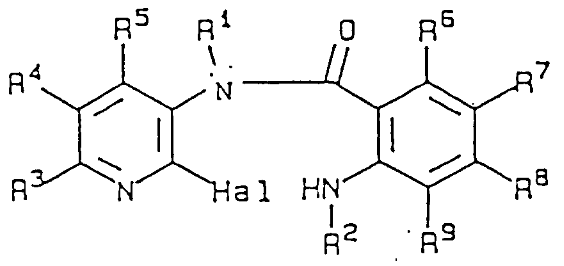

(II)

worin Hal ein Halogenatom ist und $R^1$ bis $R^9$ die gleichen Identitäten wie die entsprechenden Substituenten in der resultierenden Verbindung von Anspruch 4 besitzen;
B) das Reagierenlassen eines Isatinsäure-anhydrids der Formel

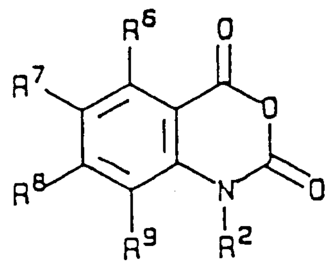

(III)

mit einem 2-Halogen-3-aminopyridin der Formel

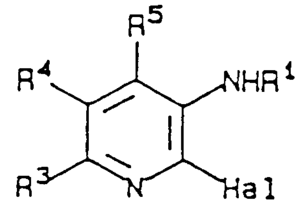

(IV)

worin $R^2$ bis $R^9$ die gleichen Identitäten wie die entsprechenden Substituenten in der resultierenden Verbindung von Anspruch 4 besitzen und Hal ein Halogenatom ist; oder

C) zwecks Herstellung einer Verbindung von Anspruch 4, worin der Substituent in der 5-Stellung ein anderer als Wasserstoff ist, das Reagierenlassen eines 5-Alkalimetall-Derivats einer Verbindung, welche der gewünschten Verbindung von Anspruch 4 entspricht, mit einer Verbindung der Formel $R^{1'}Y$, worin $R^{1'}$ dem 5-Substituenten der gewünschten Verbindung von Anspruch 4 entspricht und Y eine Abgangsgruppe ist, und die anschließende Reindarstellung der aus dem Verfahren A, B oder C resultierenden Verbindung von Anspruch 4 als solche oder als ein pharmazeutisch brauchbares Säureadditionssalz.

**6.** Verfahren zur Herstellung einer pharmazeutischen Zusammensetzung, welches das Mischen einer Verbindung gemäß Anspruch 4 mit einem pharmazeutisch brauchbaren Carrier oder Hilfsstoff umfaßt.

**Revendications**

**1.** Utilisation d'un composé de formule I

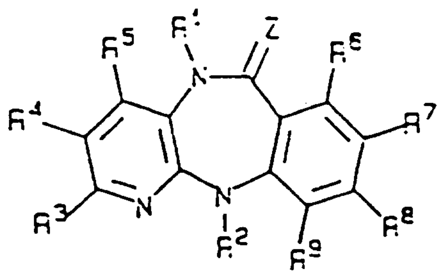

(I)

dans laquelle

Z est l'oxygène ou le soufre;

$R^1$ est l'hydrogène, un groupe alkyle ou fluoroalkyle de 1 à 4 atomes de carbone, cyclopropyle, alcényle ou alcynyle de 3 à 4 atomes de carbone, 2-halogénopropén-1-yle, arylméthyle (dans lequel la partie aryle est phényle ou thiényle, qui est non substitué ou substitué par méthyle, méthoxy ou halogène), acétyle, ou alcoxyalkyle ou alkylthioalkyle de 2 à 3 atomes de carbone;

$R^2$ est un groupe alkyle ou fluoroalkyle de 1 à 4 atomes de carbone, cycloalkyle de 3 à 5 atomes de carbone, alcényle ou alcynyle de 2 à 4 atomes de carbone, alcoxyalkyle ou alkylthioalkyle de 2 à 3 atomes de carbone, alcanoyle de 2 à 3 atomes de carbone, hydroxyalkyle de 2 à 4 atomes de carbone, arylméthyle (dans lequel la partie aryle est phényle, thiényle ou furanyle, qui est non substitué ou substitué par alkyle ou alcoxy de 1 à 3 atomes de carbone, hydroxyle ou halogène), phényle (qui est non substitué ou substitué par alkyle ou alcoxy de 1 à 3 atomes de carbone, halogène ou hydroxyle) ou alcoxycarbonylméthyle dans lequel la partie alcoxy contient 1 à 5 atomes de carbone;

$R^3$, $R^4$ et $R^5$ sont chacun indépendamment l'hydrogène ou un groupe alkyle de 1 à 3 atomes de carbone, à condition que l'un au moins de ces substituants soit l'hydrogène; ou bien

l'un des substituants $R^3$, $R^4$ et $R^5$ est un groupe butyle, alcanoyle de 2 à 4 atomes de carbone, alcoxycarbonyle de 2 à 4 atomes de carbone, hydroxyalkyle de 1 à 4 atomes de carbone, alcoxycarbonylalkyle dans lequel les parties alcoxy et alkyle contiennent chacune 1 à 2 atomes de carbone, halogène, trihalogénométhyle, hydroxyle, alcoxy de 1 à 3 atomes de carbone, alkylthio de 1 à 3 atomes de carbone, alcanoyloxy de 2 à 3 atomes de carbone, alcanoylamino de 1 à 3 atomes de carbone, aminoalkyle de 1 à 3 atomes de carbone, mono- ou dialkylamino dans lequel chaque partie

alkyle contient 1 à 2 atomes de carbone, carboxyalkyle de 2 à 3 atomes de carbone, cyano, nitro, carboxyle, carbamyle, amino, azido, mono- ou dialkylaminoalkyle dans lequel chaque partie alkyle contient 1 à 2 atomes de carbone, à condition que les deux substituants restants soient l'hydrogène ou un groupe méthyle; ou bien

lorsque Z est l'oxygène, l'un des substituants $R^3$, $R^4$ et $R^5$ est un groupe alkylsulfinyle ou alkylsulfonyle de 1 à 3 atomes de carbone à condition que les deux substituants restants soient l'hydrogène ou un groupe méthyle; et

$R^6$, $R^7$, $R^8$ et $R^9$ sont l'hydrogène; ou bien

l'un des substituants $R^6$, $R^7$, $R^8$ et $R^9$ est un groupe alkyle de 1 à 4 atomes de carbone, alcanoyle de 2 à 4 atomes de carbone, alcoxycarbonyle de 2 à 4 atomes de carbone, hydroxyalkyle de 1 à 4 atomes de carbone, alcoxycarbonylalkyle dans lequel les parties alcoxy et alkyle contiennent chacune 1 à 2 atomes de carbone, halogène, trihalogénométhyle, hydroxyle, alcoxy de 1 à 3 atomes de carbone, alkylthio de 1 à 3 atomes de carbone, alcanoyloxy de 2 à 3 atomes de carbone, alcanoylamino de 1 à 3 atomes de carbone, aminoalkyle de 1 à 3 atomes de carbone, mono- ou dialkylamino dans lequel chaque partie alkyle contient 1 à 2 atomes de carbone, carboxyalkyle de 2 à 3 atomes de carbone, cyano, nitro, carboxyle, carbamyle, amino, azido, mono- ou dialkylaminoalkyle dans lequel chaque partie alkyle contient 1 à 2 atomes de carbone, et les trois substituants restants sont l'hydrogène ou deux des trois substituants restants sont l'hydrogène et l'un est un méthyle, éthyle ou halogène;

ou d'un sel d'addition d'acide pharmaceutiquement acceptable de celui-ci

dans la préparation d'une composition pharmaceutique pour la prophylaxie ou le traitement d'un humain exposé à ou infecté par VIH-1.

**2.** Utilisation selon la revendication 1, dans laquelle, dans le composé de formule I,

Z est l'oxygène ou le soufre;

$R^1$ est l'hydrogène, un groupe alkyle de 1 à 3 atomes de carbone, allyle, propargyle, 2-halogénopropén-1-yle, méthoxyméthyle ou méthylthiométhyle;

$R^2$ est un groupe alkyle de 1 à 4 atomes de carbone, cycloalkyle de 3 à 4 atomes de carbone, alcényle ou alcynyle de 2 à 4 atomes de carbone, alcoxyalkyle ou alkylthioalkyle de 2 à 3 atomes de carbone, alcanoyle de 2 à 3 atomes de carbone, hydroxyalkyle de 2 à 4 atomes de carbone, arylméthyle (dans lequel la partie aryle est phényle ou thiényle, qui est non substitué ou substitué par méthyle ou méthoxy ou halogène) ou alcoxycarbonylméthyle dans lequel la partie alcoxy contient 1 à 4 atomes de carbone;

$R^3$, $R^4$ et $R^5$ sont chacun indépendamment l'hydrogène ou un groupe méthyle, à condition que l'un au moins de ces substituants soit l'hydrogène, ou bien $R^5$ est un groupe éthyle, propyle ou butyle et les deux substituants restants sont l'hydrogène;

$R^6$ est choisi parmi l'hydrogène, un groupe méthyle et éthyle mais peut avoir seulement l'une des deux significations citées en dernier lieu lorsque $R^7$ est l'hydrogène ou un groupe méthyle;

$R^7$ et $R^8$ sont choisis parmi l'hydrogène, un groupe méthyle ou halogène, ou bien

$R^7$ est un groupe alkyle de 1 à 2 atomes de carbone, acétyle, hydroxyalkyle de 1 à 2 atomes de carbone, alcoxycarbonyle de 2 à 3 atomes de carbone, alcoxycarbonylalkyle, dans lequel les parties alcoxy et alkyle contiennent chacune 1 à 2 atomes de carbone, halogène, trifluorométhyle, hydroxyle, alcoxy de 1 à 2 atomes de carbone, alkylthio de 1 à 2 atomes de carbone, acétyloxy, alcanoylamino de 1 à 2 atomes de carbone ou cyano, et $R^8$ est l'hydrogène; ou bien

$R^8$ est un groupe alkyle de 1 à 2 atomes de carbone, acétyle, hydroxyalkyle de 1 à 2 atomes de carbone, alcoxycarbonyle de 2 à 3 atomes de carbone, alcoxycarbonylalkyle, dans lequel les parties alcoxy et alkyle contiennent chacune 1 à 2 atomes de carbone, halogène, trifluorométhyle, hydroxyle, alcoxy de 1 à 2 atomes de carbone, alkylthio de 1 à 2 atomes de carbone, alcanoylamino de 1 à 2 atomes de carbone ou cyano, et $R^7$ est l'hydrogène; et

$R^9$ est choisi parmi l'hydrogène et un groupe méthyle mais ne peut avoir la signification citée en dernier lieu que lorsque $R^8$ est l'hydrogène ou un groupe méthyle.

**3.** Utilisation selon la revendication 1, dans laquelle, dans le composé de formule I,

Z est l'oxygène ou le soufre;

$R^1$ est l'hydrogène, un groupe 2-halogéno-2-propén-1-yle ou alkyle de 1 à 3 atomes de carbone;

$R^2$ est un groupe alkyle de 1 à 4 atomes de carbone ou cycloallcyle de 3 à 4 atomes de carbone; et

$R^3$ à $R^9$ sont chacun l'hydrogène.

**4.** Composé choisi dans le groupe consistant en les suivants:
a) 5-(2-chloro-2-propényl)-11-éthyl-5,11-dihydro-6H-pyrido[2,3-b][1,4]benzodiazépin-6-one;
b) 2,5,8,11-tétraméthyl-5,11-dihydro-6H-pyrido[2,3-b][1,4]benzodiazépin-6-one;
c) 5,11-diéthyl-10-méthyl-5,11-dihydro-6H-pyrido[2,3-b][1,4]benzodiazépin-6-one;
d) 5,8-diméthyl-11-éthyl-5,11-dihydro-6H-pyrido[2,3-b][1,4]benzodiazépin-6-one;
e) 2,5,10,11-tétraméthyl-5,11-dihydro-6H-pyrido[2,3-b][1,4]benzodiazépin-6-one;
f) [11-[5,11-dihydro-6-oxo-6H-pyrido[2,3-b][1,4]benzodiazépin-11-yl]]acétate de tertiobutyle;
g) 2-chloro-5-éthyl-11-méthyl-5,11-dihydro-6H-pyrido[2,3-b][1,4]benzodiazépin-6-one; et
h) 5,8,11-triméthyl-5,11-dihydro-6H-pyrido[2,3-b][1,4]benzodiazépin-6-one;
et leurs sels d'addition d'acide pharmaceutiquement acceptables.

**5.** Procédé de préparation d'un composé selon la revendication 4, qui comprend la mise en oeuvre de l'un des procédés suivants:
A) cyclisation d'un composé de formule

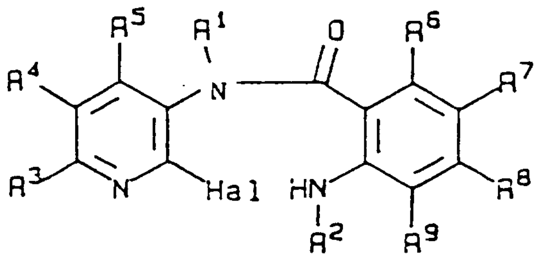

(II)

dans laquelle Hal est un atome d'halogène et $R^1$ à $R^9$ ont les mêmes significations que les substituants correspondants dans le composé résultant selon la revendication 4;
B) réaction d'un acide isatoïque de formule

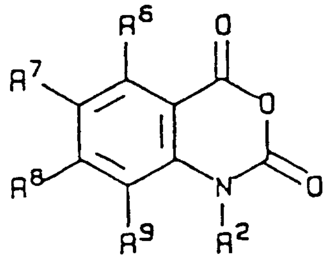

(III)

avec une 2-halogéno-3-aminopyridine de formule

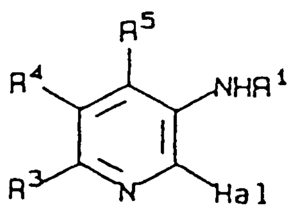

(IV)

dans lesquelles $R^2$ à $R^9$ ont les mêmes significations que les substituants correspondants dans le composé résultant selon la revendication 4, et Hal est un atome d'halogène; ou
C) pour produire un composé selon la revendication 4 dans lequel le substituant en position 5 n'est pas l'hydrogène, réaction d'un dérivé 5-métal alcalin d'un composé correspondant au composé voulu selon la revendication 4 avec un composé de formule $R^{1'}Y$ dans laquelle $R^{1'}$ correspond au substituant en 5 du composé voulu selon la revendication 4 et Y est un groupe partant,

puis isolement du composé résultant selon la revendication 4 du procédé A, B ou C, en l'état ou sous forme d'un sel d'addition d'acide pharmaceutiquement acceptable.

**6.** Procédé de préparation d'une composition pharmaceutique qui comprend le mélange d'un composé selon la revendication 4 avec un véhicule ou excipient pharmaceutiquement acceptable.